(19) **Europäisches Patentamt**

**European Patent Office**

**Office européen des brevets**

(11) Veröffentlichungsnummer: **0 240 750 B1**

(12) **EUROPÄISCHE PATENTSCHRIFT**

(45) Veröffentlichungstag der Patentschrift: **13.05.92**

(51) Int. Cl.⁵: **A61L 2/04**

(21) Anmeldenummer: **87103313.0**

(22) Anmeldetag: **09.03.87**

(54) **Verfahren zum Pasteurisieren von Plasmaproteinen und Plasmaproteinfraktionen.**

(30) Priorität: **18.03.86 AT 719/86**

(43) Veröffentlichungstag der Anmeldung:
**14.10.87 Patentblatt 87/42**

(45) Bekanntmachung des Hinweises auf die
Patenterteilung:
**13.05.92 Patentblatt 92/20**

(84) Benannte Vertragsstaaten:
**AT BE CH DE ES FR GB GR IT LI LU NL SE**

(56) Entgegenhaltungen:
**EP-A- 0 144 709**

(73) Patentinhaber: **Heilmittelwerke Wien Gesellschaft m.b.H.
Doerenkampgasse 4
A-1100 Wien(AT)**

(72) Erfinder: **Doleschel, Walter, Prof.Dr.
Sonnleitensteig 7
A-1190 Wien(AT)**
Erfinder: **Doleschel, Walter Norbert, Dr.
Siolygasse 18-22/3a
A-1190 Wien(AT)**
Erfinder: **Kaltschmid, Helmut, Dr.
Dammgasse 11
A-2540 Bad Vöslau(AT)**

(74) Vertreter: **Kunz, Ekkehard, Dr.
Chemie Holding AG Patentwesen St. Peter-
Strasse 25
A-4021 Linz(AT)**

**Beschreibung**

Die Erfindung betrifft ein neues Verfahren zum Pasteurisieren von Plasmaproteinen und Plasmaproteinfraktionen ohne wesentliche Beeinträchtigung von deren biologischer Aktivität.

Die Verabreichung von Plasmaproteinen oder Plasmaproteinfraktionen aus Humanplasma an Patienten stellt bei vielen Krankheitsfällen eine unumgängliche Behandlungsmethode dar, die aber mit dem Risiko der Übertragung von viralen Infektionskrankheiten verbunden ist. Obwohl dieses Risiko durch sorgfältige Spenderauswahl und eingehende Testung vermindert werden kann, ist bei der laufenden Gabe von Plasmaproteinen, beispielsweise an hämophile Patienten, die Infektionsgefahr dennoch nicht zu übersehen.

Seit einiger Zeit sind daher Bestrebungen im Gange, das Infektionsrisiko durch eine Virusinaktivierung, die vor allem durch Hitzebehandlung der Plasmaproteine oder einzelner ihrer Fraktionen erreicht werden kann, auszuschalten. Dem steht entgegen, daß sich zwar Albumin und hitzebeständige alpha- und beta-Globuline unter gewissen Voraussetzungen in wäßriger Lösung pasteurisieren lassen, während andere ausgewählte Plasmaproteinfraktionen, wie Immunglobuline oder Gerinnungsfaktoren, hier insbesondere Konzentrate der Antihämophiliefaktoren, ihre biologische Aktivität entweder gänzlich oder größtenteils verlieren.

Zur Vermeidung dieser Schwierigkeiten wurde bereits mehrmals vorgeschlagen, Plasmaproteine in wäßriger Lösung durch verschiedene Zusätze wie Aminosäuren, Saccharide, Zuckeralkohole, Ca-Ionen, Kalium- oder Ammoniumcitrat oder Salze von Carbonsäuren und Hydroxycarbonsäuren gegen Hitzeeinwirkung zu stabilisieren (vgl. u.a. US-PSen 4 297 344, 4 440 679,4 327 086, 4 446 134, DEOSen 3 237 512, 3 330 770 und PCT-Anmeldung WO 83/04027), jedoch müssen bei dieser Methode empfindliche Verluste der biologischen Aktivität in Kauf genommen werden.

In der EP-A-110 407 ist ein mehrstufiges Verfahren zur Entfernung von Hepatitis B Viren und Hepatitis-non-A-non-B Viren in biologischem Material beschrieben. Dieses Verfahren umfaßt in einem Teilschritt unter anderem auch eine Hitzebehandlung des biologischen Materials, bei dem nach den Angaben in der Druckschrift bei Pasteurisationstemperaturen von 55 - 70° C nur ein Aktivitätsverlust von 10 - 25 % beobachtet werden soll. Dieses Trockenerhitzungsverfahren hat aber auf jeden Fall den Nachteil, daß die Wärmeübertragung vor allem bei größeren Mengen an biologischem Material sehr ungleichmäßig ist, wodurch es einerseits durch lokale Überhitzung zur Schädigung des biologischen Materials und andererseits an Stellen geringer Wärmeübertragung zu mangelnder Virusinaktivierung kommen kann.

Aus der EP-A-112 563 und der PCT/WO 83/04371 sind Verfahren zur Beseitigung von infektiösen Viren und pyrogenen Stoffen ausschließlich mit Lipidstruktur bekannt, bei denen die Lipidhülle dieser Erreger durch extraktive Behandlung mit lipophilen Lösungsmitteln, vorzugsweise mit chlorierten Koh-lenwasserstoffen, bei Raumtemperatur zerstört wird, was zu deren Inaktivierung führt. In der EP-A-112 563 wird aber zur Vermeidung von Schädigungen der Plasmaproteine ausdrücklich von einer Hitzebehandlung abgeraten, sodaß nur Erreger mit Lipidstruktur beseitigt werden können, während andere Arten von Erregern nach dieser Methode einer Inaktivierung nicht zugänglich sind.

Schließlich wird in der US-PS 4 490 361 vorgeschlagen, Suspensionen von getrockneten Plasmaproteinen in organischen Lösungsmitteln zur Virusinaktivierung einer Hitzebehandlung zu unterwerfen. Als geeignete Lösungsmittel sind in der Druckschrift flüssige Alkane, wie Hexan oder Heptan, Ketone, wie Aceton oder Diethylketon oder perfluorierte Stoffe, wie Perfluortripropylamin, genannt. Obwohl in Suspensionen eine gute und gleichmäßige Wärmeübertragung gewährleistet ist, kann das Verfahren gemäß US-PS-4 490 361 nicht restlos befriedigen, da immer noch beachtliche Aktivitätsverluste hingenommen werden müssen. So behält ein lyophilisiertes AHF-Präparat nach zehnstündigem Erhitzen in Aceton, Hexan oder Perfluortripropylamin in dem für eine wirkungsvolle Pasteurisation aber an der Untergrenze liegenden Temperaturbereich von 60° C beispielsweise nur zwischen 59,7 bis 70,7 % seiner ursprünglichen biologischen Aktivität.

Es besteht daher nach wie vor Bedarf nach verbesserten Pasteurisationsverfahren für Plasmaproteine, wobei die Aufgabe darin besteht, neben einer guten, gleichmäßigen Wärmeübertragung und sicheren Virusinaktivierung auch eine möglichst weitgehende Erhaltung der biologischen Aktivität sicherzustellen. Außerdem soll das Verfahren von seinem Aufwand her wirtschaftlich und einfach durchzuführen sein.

Es wurde nun überraschenderweise gefunden, daß Glycerinester von gesättigten und ungesättigten Fettsäuren, wie sie beispielsweise in biologischen Ölen, verflüssigten Fetten und teilweise oder völlig hydrierten Ölen vorkommen, ein ausgezeichnetes Pasteurisationsmedium darstellen, in dem die Hitzebehandlung zur Virusinaktivierung von Plasmaproteinen und Plasmaproteinfraktionen unter weitgehender Erhaltung der biologischen Aktivität auf einfache Weise durchgeführt werden kann.

Gegenstand der Erfindung ist demnach ein Verfahren zum Pasteurisieren von getrockneten Plasmaproteinen und Plasmaproteinfraktionen durch Hitzebehandlung von deren Suspensionen in einem flüssigen organischen Wärmeüberträger ohne wesentliche Beeinträchtigung von deren biologischer Aktivität, wobei das Verfahren dadurch gekennzeichnet ist, daß man die Hitzebehandlung in bei Pasteurisationstemperatur flüssigen symmetrischen oder gemischten Glycerinestern von gesättigten oder einfach oder mehrfach ungesättigten Fettsäuren mit 4 - 22 Kohlenstoffatomen oder Gemischen dieser Ester als inertem Wärmeüberträger bei einem maximalen Wassergehalt der Suspension von 1 Gew% und Temperaturen von 50 - 120°C durchführt.

Die für das erfindungsgemäße Verfahren als Wärmeüberträger verwendeten Glycerinester, in denen die Plasmaproteine und Plasmaproteinfraktionen zur Hitzebehandlung suspendiert werden, können synthetischer Natur sein. Bevorzugt stammen die Glycerinester jedoch aus natürlichen pflanzlichen oder tierischen Quellen und sind als solche biologische Öle, verflüssigte Fette oder halbsynthetische Produkte, wie teilweise oder völlig hydrierte Öle.

Die zur Pasteurisation besonders geeigneten pflanzlichen und tierischen Fette und Öle bestehen im wesentlichen aus gemischten Glycerinestern höherer Fettsäuren. Es können dies beispielsweise Glycerinester von gesättigten Fettsäuren mit der allgemeinen Formel $C_nH_{2n+1}$ COOH, worin n eine ganze Zahl von 4 - 22 bedeutet, von einfach ungesättigten Fettsäuren der allgemeinen Formel $C_nH_{2n-1}$ COOH, worin n eine ganze Zahl von 16 - 22 bedeutet, oder von mehrfach ungesättigten Fettsäuren mit Summenformeln von $C_{19}H_{32}O_2$ bis $C_{22}H_{34}O_2$ sein, wobei die Zusammensetzung der Fettsäuren, mit denen das Glycerin verestert ist, keine Rolle spielt.

Häufig vorkommende Fettsäuren in diesen natürlichen Glycerinestern sind die Palmitinsäure ($C_{15}H_{31}$ COOH), die Stearinsäure ($C_{17}H_{35}$ COOH) und die Ölsäure ($C_{17}H_{33}$ COOH). Daneben finden sich in natürlichen Glycerinestern noch die Laurinsäure, Buttersäure, die ungesättigte Physetölsäure, die stark ungesättigte Linolsäure und in geringerer Konzentration auch andere Fettsäuren, wie Capronsäure, Caprinsäure, Caprylsäure, Arachinsäure, Linolensäure und dergleichen.

Neben synthetischen und natürlichen Glycerinestern eignen sich für das Pasteurisationsverfahren auch durch die sogenannte Fetthärtung (Hydrierung) hergestellte halbsynthetische Produkte. Diese entstehen durch katalytische Anlagerung von Wasserstoff an ungesättigte Fettsäuren in den in natürlichen Fetten und Ölen vorkommenden Glycerinestern, wobei beispielsweise die ungesättigte Ölsäure in die gesättigte Stearinsäure und die Ricinolsäure in die Hydroxystearinsäure übergeführt wird.

Die natürlichen und halbsynthetischen Fette und Öle sind in der Regel nicht aus symmetrischen Estern mit gleichen Fettsäurekomponenten, sondern aus gemischten Estern von Glycerin und Fettsäuren aufgebaut. Frische Fette und Öle sind stets Neutralfette, in denen alle drei OH-Gruppen des Glycerins mit Fettsäuren verestert sind.

Beispiele für Glycerinester der oben angegebenen Zusammensetzung aus pflanzlichen Quellen sind Maiskeimöl, Sonnenblumenkernöl, Olivenöl, Weizenkeimöl, Rapsöl, Sojaöl, Distelöl, Kernöle aus anderen Quellen und dergleichen.

Geeignete Glycerinester aus tierischen Quellen sind Milchfette oder Gewebsfette, beispielsweise die in Butter oder Butterfett vorkommenden Fette, die im wesentlichen aus Butyrodiolein, Butyropalmitolein, Oleodipalmitin, Palmitodistearin, Stearodipalmitin, Tristearin bestehen, Schweineschmalz, welches aus reinem Schweinefett mit einem Wassergehalt von weniger als 0,3 % besteht und vorwiegend aus gemischten Glycerinestern der Ölsäure, Palmitinsäure und Stearinsäure aufgebaut ist, oder Rindertalg und dergleichen.

Geeignete halbsynthetische, durch Fetthärtung erhaltene Produkte, die als Wärmeüberträger für das erfindungsgemäße Verfahren vorgesehen sind, sind Speisefette oder Margarinen unterschiedlicher Zusammensetzung und Herkunft.

In den meisten Fällen können die bei der Pasteurisation als Wärmeüberträger verwendeten Glycerinester ohne weitere Vorbehandlung in der Form eingesetzt werden, wie diese als handelsübliche Fette und Öle von der Lebensmittelindustrie angeboten werden. Üblicherweise vorhandene Zusätze in geringen Mengen, wie Mineralsalze, Vitamine, Aminosäuren oder Emulgiermittel, stören beim erfindungsgemäßen Verfahren nicht. Es muß jedoch darauf geachtet werden, daß der Wassergehalt in der Suspension höchstens 1 Gew% beträgt. Ein höherer Wassergehalt in der Suspension der Plasmaproteine und Plasmaproteinfraktionen kann zu stärkerer Denaturierung der Proteine und damit zu einem erheblichen Verlust ihrer biologischen Aktivität führen. So werden beispielsweise bei der Hitzebehandlung von antihämophilem Plasma (AHP) in einer Suspension von Schweineschmalz, die 2 Gew% Wasser enthält nach einer Pasteurisationszeit von 21,5 Stunden bei 60°C Produkte erhalten, deren Gerinnungsaktivität nur mehr 20 % der Ausgangsaktivität vor der Pasteurisation beträgt.

Vorteilhafterweise beträgt der Wassergehalt in der zu pasteurisierenden Suspension der Plasmaproteine oder deren Fraktionen höchstens 0,5 Gew%.

Fette und Öle, die von ihrer Natur oder Herstellung her einen höheren Wassergehalt aufweisen, werden zweckmäßigerweise vor der Verwendung zur Herabsetzung des Wassergehaltes getrocknet, beispielsweise durch Zentrifugation der verflüssigten Fette oder durch Behandlung mit anorganischen, wasserfreien, hygroskopischen Substanzen wie z.B. $Na_2SO_4$ oder $CaCl_2$.

Dabei wurde festgestellt, daß der Zusatz von Ca-Salzen zur Suspension in vielen Fällen die Erhaltung der biologischen Aktivität der Plasmaproteine während der Pasteurisation besonders günstig beeinflußt. Insbesondere bei bestimmten Gerinnungsfaktoren, wie beispielsweise Faktor VIII, Faktor IX und Fibrinogen, die in Verbindung mit Ca-Ionen wirksam sind, konnte durch Zusatz von Ca-Salzen eine nahezu 100 % Stabilität ihrer Gerinnungsaktivität nach der Pasteurisation festgestellt werden. In einer bevorzugten Ausführungsform des erfindungsgemäßen Verfahrens wird daher die Hitzebehandlung der Plasmaproteine in Anwesenheit von Ca-Salzen in der Suspension in einer Menge von 10 bis 50 mMol pro Liter durchgeführt.

Das erfindungsgemäße Verfahren kann auf Plasmaproteine oder Plasmaproteinfraktionen, die in bekannter Weise aus menschlichem Blutplasma isoliert wurden, angewandt werden, beispielsweise Antithrombin III, Faktor VIII, Fibrinogen, die Proteine des Prothrombinkomplexes, wie die Faktoren II, VII, IX und X, Protein C, Protein S, Immunglobuline, gefriergetrocknetes Frischplasma und dergleichen. Die Herstellung dieser Plasmaproteine und Plasmaproteinfraktionen ist bekannt und in der einschlägigen Literatur mehrfach beschrieben.

Für das erfindungsgemäße Pasteurisationsverfahren werden die Plasmaproteine oder Plasmaproteinfraktionen in getrockneter Form eingesetzt, wie sie beispielsweise durch Lyophilisation, Sprühtrocknung oder andere schonende Trocknungsmethoden aus den bei der Fraktionierung anfallenden wäßrigen Lösungen der Plasmaproteine üblicherweise erhalten werden. Diese werden sodann in feinteilige Form gebracht und im jeweils für die Hitzebehandlung gewählten Wärmeüberträger suspendiert. Werden als Wärmeüberträger Glycerinester gewählt, die bei Raumtemperatur in festem Aggregatzustand oder in Form von zähflüssigen Ölen vorliegen, ist es empfehlenswert, den Wärmeüberträger zur Herstellung der Suspension vor der Zugabe der Plasmaproteine durch Erwärmen in einen leichtflüssigen Aggregatzustand überzuführen. Die Plasmaproteine sind in den beim erfindungsgemäßen Verfahren vorgesehenen Glycerinestern nicht löslich. Temperatur und Behandlungsdauer der erfindungsgemäßen Pasteurisation richtet sich nach den für die Pasteurisation von Plasmafraktionen gebräuchlichen Werten.

Im allgemeinen können Temperaturen von 50 - 120° C und Pasteurisationszeiten von 2 Stunden bis zu mehreren Tagen Anwendung finden, es sind aber auch andere Varianten, wie z.B. schockartiges Erhitzen von nur wenigen Sekunden möglich. Die Dauer der Hitzebehandlung hängt von der gewählten Temperatur ab. Als besonders günstiger Bereich haben sich für eine schonende Behandlung der Plasmaproteine und sichere Virusinaktivierung Temperaturen von 60 - 70° C und eine Pasteurisationsdauer von 10 bis 48 Stunden erwiesen.

Natürlich nimmt die Abnahme der biologischen Aktivität mit der Dauer der Behandlung und höheren Temperaturen in der Regel zu. Im Vergleich zu den Verfahren nach dem Stand der Technik ist das erfindungsgemäße Verfahren überraschenderweise aber sehr schonend. So bleiben z.B. bei der erfindungsgemäßen Pasteurisation verschiedener Produktionschargen von Faktor VIII-Kon zentraten bei einer Behandlungsdauer von 16 Stunden und einer Behandlungstemperatur von 60° C 90 - 100 % der ursprünglichen AHF-Aktivität dieser Präparate erhalten.

Die Aufarbeitung und Reindarstellung der Plasmaproteine und Plasmaproteinfraktionen nach beendeter erfindungsgemäßer Pasteurisation gestaltet sich sehr einfach. Der verwendete Wärmeüberträger kann durch Abfiltrieren oder Abnutschen der Plasmaproteine entfernt werden, wobei es in vielen Fällen zweckmäßig ist, die Abtrennung des Wärmeüberträgers unmittelbar nach Beendigung der Hitzebehandlung durchzuführen, bevor dieser erstarrt oder dickflüssig wird. Der Wärmeüberträger kann aber auch durch Behandeln mit organischen Lösungsmitteln entfernt werden. So ist es beispielsweise in Fällen, in denen der Wärmeüberträger vor der anschließenden Verarbeitung der Plasmaproteine oder zum Zwecke der Lagerung völlig entfernt werden soll, empfehlenswert, die Plasmaproteine mit einem wasserfreien organischen Lösungsmittel, das leicht flüchtig ist und in dem der verwendete Wärmeüberträger gut löslich ist, beispielsweise mit n-Hexan, Petrolether, Leichtbenzin und dergleichen auszuwaschen.

Die restlose Entfernung des leicht flüchtigen Waschmittels kann dann zweckmäßigerweise im Vakuum, gegebenenfalls unter gelindem Erwärmen, erfolgen.

Das erfindungsgemäße Pasteurisationsverfahren hat neben der überraschend schonenden Behandlung der Plasmaproteine unter größtmöglicher Erhaltung ihrer biologischen Aktivität den Vorteil, daß die verwendeten Wärmeüberträger aus natürlichen, biologischen Quellen gewonnen werden können und somit keinerlei Toxizitätsprobleme auftreten. Außerdem ist das Verfahren einfach durchzuführen und ermöglicht die Hitzeinaktivierung von Krankheitserregern in leichtzugänglichen und günstig erhältlichen Wärmeüberträgern unter äußerst wirtschaftlichen Bedingungen.

Die folgenden Beispiele erläutern die Erfindung näher:

Beispiel 1:

Von drei nach bekannten Methoden hergestellten Produktionschargen von Faktor VIII und einer Charge von gefriergetrocknetem Frischplasma (AHP) wurden je 1 g der gefriergetrockneten Präparationen mit je 10 g eines im Handel erhältlichen, getrockneten Sonnenblumenkernöls bei 60° C vermischt (Das Öl wurde mit wasserfreiem $CaCl_2$-Pulver 2 Stunden bei 60° C getrocknet und vor dem Vermischen mit den Proteinfraktionen durch Dekantieren vom $CaCl_2$ getrennt). Die Protein-Öl-Suspensionen wurden 16 Stunden im Brutschrank bei 60° C +-0,2° C pasteurisiert. Nach beendeter Pasteurisation wurde das noch warme Öl über eine Nutsche abgesaugt, das Präparat viermal mit n-Hexan nachgewaschen und zuerst an der Luft und anschließend im Vakuum getrocknet.

Die biologische Faktor VIII Aktivität betrug die folgenden Prozentsätze vom Ausgangswert vor der Pasteurisation:

| | |
|---|---|
| Charge I | 89,5 % |
| Charge II | 100,0 % |
| Charge III | 93,3 % |
| AHP-Charge | 90,1 % |

Beispiel 2:

Das Beispiel 1 wurde mit Proben aus den gleichen Chargen wiederholt, nur wurde eine andere Marke eines im Handel erhältlichen Sonnenblumenkernöls verwendet, welches wie in Beispiel 1 getrocknet wurde. Die biologische Faktor VIII-Aktivität betrug die folgenden Prozentsätze vom Ausgangswert vor der Pasteurisation:

| | |
|---|---|
| Charge I | 91,0 % |
| Charge II | 98,3 % |
| Charge III | 92,7 % |
| AHP-Charge | 89,0 % |

Aus dem Vergleich von Beispiel 1 und 2 sieht man, daß die Werte für eine bestimmte Charge unabhängig von der Marke des verwendeten Sonnenblumenkernöls innerhalb der Meßgenauigkeit der Aktivitätsbestimmung bei biologischen Proben liegt.

Beispiel 3:

5 g lyophilisiertes Frischplasma (AHP) wurde in 5 Portionen, wie in Beispiel 1 mit wasserfreiem Maiskeimöl bei 60° C pasteurisiert. Nach 1, 2 1/2, 5, 24 und 48 Stunden wurde je eine Portion wie in Beispiel 1 aufgearbeitet und auf Faktor VIII, Faktor IX und Faktor X getestet.

Die biologische Aktivität betrug die folgenden Prozentsätze vom Ausgangswert vor der Pasteurisation:

| | F VIII | F IX | FX |
|---|---|---|---|
| 1 Stunde | 97,0 % | 95,0 % | 93,4 % |
| 2 1/2 Stunden | 95,8 % | 94,1 % | 89,0 % |
| 5 Stunden | 93,4 % | 92,3 % | 87,5 % |
| 24 Stunden | 87,2 % | 85,2 % | 80,7 % |
| 48 Stunden | 78,8 % | 79,6 % | 75,5 % |

Beispiel 4:

Das Beispiel 3 wurde wiederholt, nur wurde getrocknetes Sonnenblumenöl anstelle von Maiskeimöl verwendet.

Die gemessenen Aktivitätswerte waren:

|  | F VIII | F IX | FX |
|---|---|---|---|
| 1 Stunde | 99,7 % | 96,7 % | 93,0 % |
| 2 1/2 Stunden | 90,6 % | 92,4 % | 88,2 % |
| 5 Stunden | 84,8 % | 85,2 % | 84,5 % |
| 24 Stunden | 79,4 % | 81,4 % | 80,6 % |
| 48 Stunden | 77,7 % | 80,0 % | 78,3 % |

Beispiel 5:

Je 2 g von drei neuen Chargon von gefriergetrocknetem Faktor VIII-Präparationen wurden in 1 g-Portionen mit gehärtetem Pflanzenfett wie in Beispiel 1 bei 60° C für 2 und 16 Stunden pasteurisiert. Die Aktivitätswerte waren:

|  | 2 Stunden | 16 Stunden |
|---|---|---|
| Charge A | 97,7 % | 83,2 % |
| Charge B | 99,8 % | 91,7 % |
| Charge C | 98,3 % | 88,7 % |

Beispiel 6:

Je 1g Faktor VIII-Pulver von drei gefriergetrockneten Faktor VIII-Chargen und gefriergetrcknetem Frischplasma (AHP) wurden mit getrocknetem Schweineschmalz wie in Beispiel 1 pasteurisiert. Die Faktor VIII-Werte nach der Pasteurisation waren, bezogen auf 100 % Ausgangsaktivität

| F VIII | Charge I | 78,0 % |
|---|---|---|
|  | Charge II | 89,5 % |
|  | Charge III | 87,3 % |
| AHP |  | 90,1 % |

Beispiel 7:

2 g Faktor VIII oder gefriergetrocknetes Frischplasma (AHP) wurden in 1 g - Portionen mit je 25 g getrocknetem Schweineschmalz bei 60° C vermischt. Von diesen Portionen wurden je eine Faktor VIII- und eine AHP-Portion mit 0,5 ml $H_2O$ zu Beginn der Pasteurisation, die 21,5 Stunden dauerte, versetzt. Nach dem Aufarbeiten, das wie in Beispiel 1 erfolgte, wurden in den Faktor VIII-Proben die Faktor VIII-Werte und in den AHP-Proben die Fibrinogen und Faktor IX-Werte als Prozent der Ausgangsaktivität bestimmt. Diese betrugen für die Faktor VIII-Proben:

% Faktor VIII - Aktivität

ohne Zusatz          81,1 %
mit 0,5 ml $H_2O$      20,0 %
   Im AHP betrugen die Werte

|  | Fibrinogen | Faktor IX |
|---|---|---|
| ohne Zusatz | 96,8 % | 85,7 % |
| mit 0,5 ml $H_2O$ | 1,0 % | 21,0 % |

6

Beispiel 8:

2 g Faktor VIII oder gefriergetrocknetes Frischplasma wurden in 1 g Portionen mit je 25 g getrocknetem Schweineschmalz und 180 mg CaCl$_2$ 2H$_2$O bei 60° C versetzt und 21,5 Stunden bei 60° C pasteurisiert. Nach dem Aufarbeiten, das wie in Beispiel 1 erfolgte, wurden in den Faktor VIII-Proben die Faktor VIII-Werte und in den AHP-Proben die Fibrinogen- und Faktor IX-Werte als Prozent der Ausgangsaktivität bestimmt.

Diese betrugen für die Faktor VIII-Proben:

% Faktor VIII

ohne Zusatz          81,1 %
mit CaCl$_2$. 2H$_2$O     95,8 %
    Im AHP betrugen die Werte:

|                      | Fibrinogen | Faktor IX |
|----------------------|------------|-----------|
| ohne Zusatz          | 96,8 %     | 85,7 %    |
| mit CaCl$_2$. 2H$_2$O   | 100,0 %    | 98,4 %    |

Beispiel 9:

Trocknung des Wärmeüberträgers für die Pasteurisation:
100 g Maiskeimöl wurden mit 20 g wasserfreiem Calziumchlorid 4 h lang geschüttelt. Anschließend wurde das CaCl$_2$ abzentrifugiert.
Pasteurisation:
1 g lyophilisiertes Faktor IX/PPSB Pulver wurde in 10 g Maiskeimöl suspendiert. Die Suspension wurde 30 min auf 98° C unter Durchmischung erwärmt. Anschließend wurde die noch warme Suspension über ein Papierfilter vakuumfiltriert und der Filterkuchen mehrmals mit n-Heptan zur Entfernung des Maiskeimöls ausgewaschen.
Der Filterkuchen wurde anschließend vakuumgetrocknet.
Die Aktivitätsausbeuten an Gerinnungsfaktoren waren wie folgt (Prozentsatz der Aktivität von 100 % der Ausgangsaktivität vor Erhitzung):

| Faktor IX | 73 %   |
|-----------|--------|
| Faktor X  | 84,2 % |
| Faktor II | 84,4 % |

Aktivierte Faktoren konnten nach Erhitzung nicht nachgewiesen werden.

Beispiel 10:

Nach der gleichen Versuchsdurchführung wie unter Beispiel 9 wurde eine Erhitzung von Faktor IX/PPSB über 50 Stunden bei 60° C durchgeführt. Die Aktivitätsausbeuten an Gerinnungsfaktoren waren wie folgt. (Prozentsatz der Aktivität von 100 % Ausgangsaktivität vor Erhitzung):

| Faktor IX | 79,6 % |
|-----------|--------|
| Faktor X  | 76,9 % |
| Faktor II | 77,6 % |

Beispiel 11:

Maiskeimöl wurde wie in Beispiel 9 getrocknet.

Anstelle von Faktor IX wurde i. m. Gammaglobulin in der gleichen Versuchsanordnung wie in Beispiel 10 zur Pasteurisation eingesetzt. Die Molekulargewichtsverteilung vor und nach der Pasteurisation wurde in der Ultrazentrifuge bestimmt und ist in nachfolgender Tabelle angeführt.

| | Vor Pasteurisation | Nach Pasteurisation bei 98° (30 min) | Nach Pasteurisation bei 60° (50 h) |
|---|---|---|---|
| Spaltprodukte (ca.5 S) | 0,6 % | 0,4 % | 0,8 % |
| 7 S Gammagrlobulin | 87,6 % | 88,2 % | 86,2 % |
| 10 S Dimere | 9,0 % | 8,0 % | 8,2 % |
| >15 S Polymere | 2,8 % | 3,4 % | 4,8 % |

## Patentansprüche

1. Verfahren zum Pasteurisieren von getrockneten Plasmaproteinen und Plasmaproteinfraktionen durch Hitzebehandlung von deren Suspensionen in einem flüssigen organischen Wärmeüberträger ohne wesentliche Beeinträchtigung von deren biologischer Aktivität, dadurch gekennzeichnet, daß man die Hitzebehandlung in bei Pasteurisationstemperatur flüssigen, symmetrischen oder gemischten Glycerinestern von gesättigten oder einfach oder mehrfach ungesättigten Fettsäuren mit 4 - 22 Kohlenstoffatomen oder Gemischen dieser Ester als inertem Wärmeüberträger bei einem maximalen Wassergehalt der Suspension von 1 Gew% und bei Temperaturen von 50 -120° C durchführt.

2. Verfahren nach Anspruch 1, dadurch gekennzeichnet, daß man als Wärmeüberträger biologische Öle oder Fette pflanzlichen Ursprungs verwendet.

3. Verfahren nach Anspruch 1, dadurch gekennzeichnet, daß man als Wärmeüberträger Milch- oder Gewebsfette tierischen Ursprungs verwendet.

4. Verfahren nach Anspruch 1, dadurch gekennzeichnet, daß man als Wärmeüberträger halbsynthetische Fette verwendet, die durch Fetthärtung der in natürlichen Fetten und Ölen vorkommenden Glycerinester gewonnen wurden.

5. Verfahren nach Anspruch 1, dadurch gekennzeichnet, daß man die Hitzebehandlung bei einem Wassergehalt der Suspension von höchstens 0,5 Gew% durchführt.

6. Verfahren nach Anspruch 1, dadurch gekennzeichnet, daß man die Hitzebehandlung in Gegenwart von Ca-Salzen in der Suspension in einer Menge von 10 bis 50 mMol pro Liter durchführt.

7. Verfahren nach Anspruch 1, dadurch gekennzeichnet, daß man die Hitzebehandlung bei Temperaturen von 60 - 70° C und einer Pasteurisationszeit von 10 bis 48 Stunden durchführt.

8. Verfahren nach Anspruch 1, dadurch gekennzeichnet, daß man das Plasmaprotein oder die Plasmaproteinfraktionen nach beendetem Pasteurisieren aus der Suspension abfiltriert, mit einem wasserfreien organischen Lösungsmittel, das leichtflüchtig ist und in dem der Wärmeüberträger gut löslich ist, auswäscht und anschließend schonend trocknet.

## Claims

1. Process for the pasteurisation of dried plasma proteins and plasma protein fractions by heat treatment of their suspensions in a liquid organic heat-transfer agent without essentially impairing their biological activity, characterised in that the heat treatment is carried out in symmetrical or in mixed glycerol esters, which are liquid at the pasteurisation temperature, of saturated or singly or multiply unsaturated fatty acids having 4-22 carbon atoms, or mixtures of these esters, as the inert heat-transfer agent, with the maximum water content of the suspension being 1% by weight and the temperature being 50-120°C.

2. Process according to Claim 1, characterised in that biological oils or fats of vegetable origin are used as heat-transfer agents.

3. Process according to Claim 1, characterised in that milk fats or tissue fats of animal origin are used as heat-transfer agents.

4. Process according to Claim 1, characterised in that semisynthetic fats which have been obtained by fat hardening of the glycerol esters occurring in natural fats and oils are used as heat-transfer agents.

5. Process according to Claim 1, characterised in that the heat treatment is carried out with the water content of the suspension not exceeding 0.5% by weight.

6. Process according to Claim 1, characterised in that the heat treatment is carried out in the presence of Ca salts in the suspension in an amount of 10 to 50 mmol per litre.

7. Process according to Claim 1, characterised in that the heat treatment is carried out at temperatures of 60-70°C and with a pasteurisation time of 10 to 48 hours.

8. Process according to Claim 1, characterised in that the plasma protein or the plasma protein fractions are, after the pasteurisation is complete, removed from the suspension by filtration, washed with an anhydrous volatile organic solvent in which the heat-transfer agent is readily soluble, and then dried in a non-damaging way.

**Revendications**

1. Procédé de pasteurisation des protéines de plasma et des fractions protéiques de plasma sèches, par un traitement à la chaleur de leur suspension dans un diffuseur de chaleur organique liquide sans préjudice fondamental de leur activité biologique, caractérisé en ce qu'on réalise le traitement par la chaleur à la température de pasteurisation dans des esters glycériques liquides symétriques ou mélangés, d'acides gras saturés, simples ou plusieurs fois insaturés, de 4 à 22 atomes de carbone, ou bien des mélanges de ces esters comme diffuseur de chaleur, avec une proportion d'eau maximale dans la suspension de 1 % en masse et une température de 50 à 120°C.

2. Procédé selon la revendication 1, caractérisé en ce qu on utilise comme diffuseur de chaleur, des huiles ou matières grasses biologiques d'origine végétale.

3. Procédé selon la revendication 1, caractérisé en ce qu'on utilise comme diffuseur de chaleur des matières grasses lactiques ou de tissus d'origine animale.

4. Procédé selon la revendication 1, caractérisé en ce qu'on utilise comme diffuseur de chaleur des matières grasses semi-synthétiques obtenues par hydrogénation des esters glycériques provenant des matières grasses et huiles naturelles.

5. Procédé selon la revendication 1, caractérisé en ce qu'on conduit le traitement à la chaleur avec une proportion en eau dans la suspension au plus égale à 0,5% en masse.

6. Procédé selon la revendication 1, caractérisé en ce qu'on conduit le traitement à la chaleur en présence de sels de calcium dans la suspension selon une quantité de 10 à 50 mmoles par litre.

7. Procédé selon la revendication 1, caractérisé en ce qu'on conduit le traitement à la chaleur à une température comprise entre 60 et 70°C et une de pasteurisation de 10 à 48 heures.

8. Procédé selon la revendication 1, caractérisé en ce qu'on filtre les protéines de plasma ou les fractions protéiques de plasma, à la fin de la pasteurisation à partir de la suspension, on lavé avec un solvant organique anhydre, légèrement volatil et dans lequel le diffuseur de chaleur est très soluble, puis on sèche ensuite complètement.